(19) Europäisches Patentamt
European Patent Office
Office européen des brevets

(11) EP 1 616 569 A1

(12) EUROPEAN PATENT APPLICATION
published in accordance with Art. 158(3) EPC

(43) Date of publication:
**18.01.2006 Bulletin 2006/03**

(21) Application number: **04724784.6**

(22) Date of filing: **31.03.2004**

(51) Int Cl.:
***A61K 31/443*** *(2000.01)* ***A61K 31/575*** *(1985.01)*
***A61K 31/58*** *(1985.01)* ***A61P 11/00*** *(2000.01)*
***A61P 11/06*** *(2000.01)* *C07D 405/06 (1990.01)*
*C07J 5/00 (1974.07)* *C07J 7/00 (1974.07)*
*C07J 17/00 (1974.07)* *C07J 31/00 (1974.07)*
*C07J 71/00 (1974.07)*

(86) International application number:
**PCT/JP2004/004623**

(87) International publication number:
**WO 2004/087149 (14.10.2004 Gazette 2004/42)**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **31.03.2003 JP 2003094508**

(71) Applicant: **KYOWA HAKKO KOGYO CO., LTD.**
**Chiyoda-ku,**
**Tokyo 100-8185 (JP)**

(72) Inventors:
- **MIE, Motoya**
  **c/o Pharmaceutical Research Center**
  **Sunto-gun Shizuoka 411-8731 (JP)**
- **MANABE, H.**
  **c/o Pharmaceutical Research Center**
  **Sunto-gun Shizuoka 411-8731 (JP)**

(74) Representative: **Casalonga, Axel et al**
**BUREAU D.A. CASALONGA - JOSSE**
**Paul-Heyse-Strasse 33**
**80336 München (DE)**

# (54) MEDICINAL COMPOSITION

(57) The present invention provides a pharmaceutical composition, which comprises 7-[2-(3,5-dichloro-4-pyridyl)-1-oxoethyl]-4-methoxy-spiro[1,3-benzodioxol-2,1'-cyclopentane] or a pharmaceutically acceptable salt thereof and a steroid agent; an agent for treating and/or preventing a pulmonary disease; the agent which comprises 7-[2-(3,5-dichloro-4-pyridyl)-1-oxoethyl]-4-methoxy-spiro[1,3-benzodioxol-2,1'-cyclopentane] or a pharmaceutically acceptable salt thereof and a steroid agent which are simultaneously or separately with an interval and the like.

EP 1 616 569 A1

## Description

Technical Field

The present invention relates to a pharmaceutical composition comprising a phosphodiesterase IV inhibitor (PDE-IV inhibitor) and a steroid agent.

Background Art

It is conventionally known that combined administration of a PDE-IV inhibitor and a steroid agent is useful for obstructive pulmonary diseases such as asthma and chronic obstructive pulmonary diseases (COPD) (WO01/32127).
On the other hand, it is known that 7-[2-(3,5-dichloro-4-pyridyl)-1-oxoethyl]-4-methoxy-spiro[1,3-benzodioxo1-2,1'-cyclopentane] or a pharmaceutically acceptable salt thereof is used as a PDE-IV inhibitor (WO96/36624).

Disclosure of the Invention

An object of the present invention is to provide a pharmaceutical composition which comprises 7-[2-(3,5-dichloro-4-pyridyl)-1-oxoethyl]-4-methoxy-spiro[1,3-benzodioxol-2,1'-cyclopentane] or a pharmaceutically acceptable salt thereof and a steroid agent, and the like.
The present invention relates to the following (1) to (31):

(1) A pharmaceutical composition, which comprises (a) 7-[2-(3,5-dichloro-4-pyridyl)-1-oxoethyl]-4-methoxy-spiro[1,3-benzodioxol-2,1'-cyclopentane] represented by formula (I)

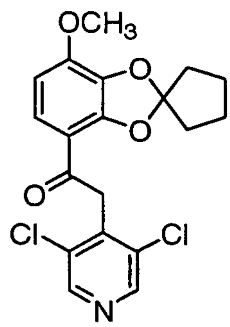

(I)

or a pharmaceutically acceptable salt thereof and (b) a steroid agent.
(2) The pharmaceutical composition according to (1), wherein the steroid agent is a compound selected from the group consisting of prednisolone, methylprednisolone, prednisone, dexamethasone, fluticasone propionate, beclometasone propionate, budesonide, flunisolide and mometasone furancarboxylate.
(3) An agent for treating and/or preventing a pulmonary disease, which comprises (a) 7-[2-(3,5-dichloro-4-pyridyl)-1-oxoethyl]-4-methoxy-spiro[1,3-benzodioxol-2,1'-cyclopentane] represented by formula (I)

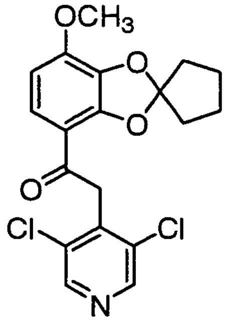

( I )

or a pharmaceutically acceptable salt thereof and (b) a steroid agent as active ingredients, wherein the active ingredients are administered simultaneously or separately with an interval.

(4) The agent for treating and/or preventing a pulmonary disease according to (3), wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

(5) The agent for treating and/or preventing a pulmonary disease according to (3) or (4), wherein the pulmonary disease is that selected from the group consisting of COPD, pulmonary emphysema, chronic bronchitis, chronic/acute respiratory distress syndrome (ARDS), acute lung injury (ALI), asthma, bronchial asthma, acute eosinophilic pneumonia and chronic eosinophilic pneumonia.

(6) The agent for treating and/or preventing a pulmonary disease according to (3) or (4), wherein the pulmonary disease is that selected from the group consisting of asthma, bronchial asthma, acute eosinophilic pneumonia and chronic eosinophilic pneumonia.

(7) The agent for treating and/or preventing a pulmonary disease according to (3) or (4), wherein the pulmonary disease is COPD.

(8) A kit which comprises (a) a first component containing 7-[2-(3,5-dichloro-4-pyridyl)-1-oxoethyl]-4-methoxy-spiro[1,3-benzodioxol-2,1'-cyclopentane] represented by formula (I)

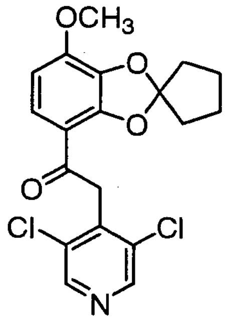

( I )

or a pharmaceutically acceptable salt thereof and (b) a second component containing a steroid agent.

(9) The kit according to (8), wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

(10) A kit for treating and/or preventing a pulmonary disease, which comprises (a) a first component containing 7-[2-(3,5-dichloro-4-pyridyl)-1-oxoethyl]-4-methoxy-spiro[1,3-benzodioxol-2,1'-cyclopentane] represented by formula (I)

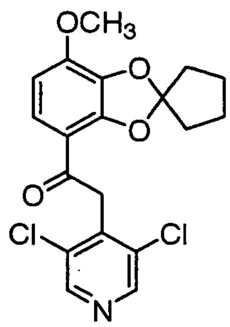

( I )

or a pharmaceutically acceptable salt thereof and (b) a second component containing a steroid agent.

(11) The kit for treating and/or preventing a pulmonary disease according to (10), wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

(12) The kit for treating and/or preventing a pulmonary disease according to (10) or (11), wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.

(13) The kit for treating and/or preventing a pulmonary disease according to (10) or (11), wherein the pulmonary disease is that selected from the group consisting of asthma, bronchial asthma, acute eosinophilic pneumonia and chronic eosinophilic pneumonia.

(14) The kit for treating and/or preventing a pulmonary disease according to (10) or (11), wherein the pulmonary disease is COPD.

(15) 7-[2-(3,5-Dichloro-4-pyridyl)-1-oxoethyl]-4-methoxy-spiro[1,3-benzodioxol-2,1'-cyclopentane] represented by formula (I)

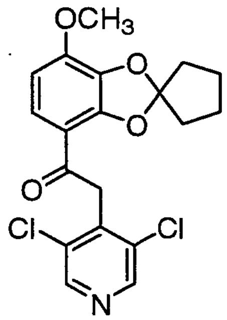

( I )

or a pharmaceutically acceptable salt thereof, which is administered with a steroid agent simultaneously or separately with an interval.

(16) 7-[2-(3,5-Dichloro-4-pyridyl)-1-oxoethyl]-4-methoxy-spiro[1,3-benzodioxol-2,1'-cyclopentane] or a pharmaceutically acceptable salt thereof according to (15), wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

(17) A pharmaceutical composition which comprises 7-[2-(3,5-dichloro-4-pyridyl)-1-oxoethyl]-4-methoxy-spiro[1,3-benzodioxol-2,1'-cyclopentane] represented by formula (I)

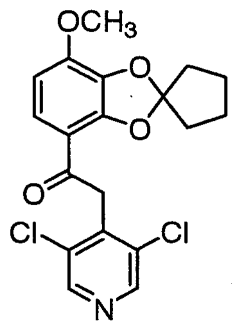

(I)

or a pharmaceutically acceptable salt thereof as a active ingredient which is administered with a steroid agent simultaneously or separately with an interval.

(18) The pharmaceutical composition according to (17), wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

(19) A method for treating and/or preventing a pulmonary disease, which comprises administering (a) 7-[2-(3,5-dichloro-4-pyridyl)-1-oxoethyl]-4-methoxy-spiro[1,3-benzodioxol-2,1'-cyclopentane] represented by formula (I)

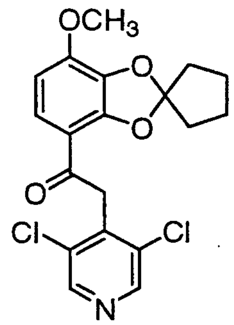

(I)

or a pharmaceutically acceptable salt thereof and (b) a steroid agent simultaneously or separately with an internal.

(20) The method for treating and/or preventing a pulmonary disease according to (19), wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

(21) The method for treating and/or preventing a pulmonary disease according to (19) or (20), wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.

(22) The method for treating and/or preventing a pulmonary disease according to (19) or (20), wherein the pulmonary disease is that selected from the group consisting of asthma, bronchial asthma, acute eosinophilic pneumonia and chronic eosinophilic pneumonia.

(23) The method for treating and/or preventing a pulmonary disease according to (19) or (20), wherein the pulmonary disease is COPD.

(24) A method for treating and/or preventing a pulmonary disease, which comprises administering the pharmaceutical composition according to (1) or (2).

(25) The method for treating and/or preventing a pulmonary disease according to (24), wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.

(26) The method for treating and/or preventing a pulmonary disease according to (24), wherein the pulmonary disease is that selected from the group consisting of asthma, bronchial asthma, acute eosinophilic pneumonia and chronic eosinophilic pneumonia.

(27) The method for treating and/or preventing a pulmonary disease according to (24), wherein the pulmonary disease is COPD.

(28) Use of (a) and (b) according to (1) or (2) for manufacture of an agent for treating and/or preventing a pulmonary disease.
(29) Use according to (28), wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.
(30) Use according to (28), wherein the pulmonary disease is that selected from the group consisting of asthma, bronchial asthma, acute eosinophilic pneumonia and chronic eosinophilic pneumonia.
(31) Use according to (28), wherein the pulmonary disease is COPD.

Hereinafter, 7-[2-(3,5-dichloro-4-pyridyl)-1-oxoethyl]-4-methoxy-spiro[1,3-benzodioxol-2,1'-cyclopentane] represented by formula (I)

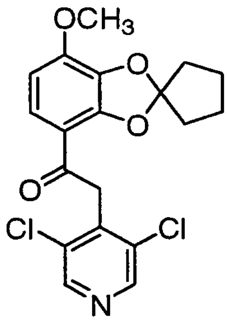

( I )

is referred to as "Compound (I)".
Pharmaceutically acceptable salts of Compound (I) include pharmaceutically acceptable acid addition salts, metal salts, ammonium salts, organic amine addition salts, amino acid addition salts and the like.
Examples of pharmaceutically acceptable acid addition salts of Compound (I) include inorganic acid salts such as a hydrochloride, a sulfate, a hydrobromide, a nitrate and a phosphate; and organic acid salts such as an acetate, a mesilate, a succinate, a maleate, a fumarate, a citrate and a tartrate. Examples of pharmaceutically acceptable metal salts include alkali metal salts such as a sodium salt and a potassium salt; alkaline earth metal salts such as a magnesium salt and a calcium salt; an aluminum salt; a zinc salt and the like. Examples of pharmaceutically acceptable ammonium salts include ammoniums salts, tetramethylammonium salts and the like. Examples of pharmaceutically acceptable organic amine addition salts include addition salts of morpholine, piperidine or the like. Examples of pharmaceutically acceptable amino acid addition salts include addition salts of glycine, phenylalanine, lysine, asparagic acid, glutamic acid or the like.
A method for preparing Compound (I) will be described below.
Compound (I) can be prepared by the method described in WO96/36624.
Among Compound (I), stereoisomers such as tautomers may be existed, however, all possible isomers and the mixture thereof can be used for the pharmaceutical composition, the agent for treating and/or preventing a pulmonary disease, the kit, the kit for treating and/or preventing a pulmonary diseases and the method for treating and/or preventing a pulmonary disease of the present invention, and Compound (I) of the present invention includes all possible isomers and a mixture thereof.
To obtain a salt of Compound (I), when Compound (I) is obtained in the form of a salt, it may be purified as it is. When Compound (I) is obtained in the free form, Compound (I) may be dissolved or suspended in a suitable solvent, followed by addition of an acid or a base to form a salt. Then, the resulting salt may be isolated and purified.
Compound (I) and a pharmaceutically acceptable salts thereof may be present in the form of an adduct with water or any one of various solvents. These adducts can also be used for the pharmaceutical composition, the agent for treating and/or preventing a pulmonary disease, the kit, the kit for treating and/or preventing a pulmonary diseases and the method for treating and/or preventing a pulmonary disease of the present invention, and the adducts are also included in Compound (I) or a pharmaceutically acceptable salts thereof.
As the steroid agent, any one of steroid derivatives or a pharmaceutically acceptable salts thereof having the function to decrease the amounts of cytokine inducing inflammation, mast cells, eosinophils and the like, and the function to inhibit migration or activation of inflammatory cells may be used. Examples of the steroid agent include prednisolones such as prednisolone represented by formula (A), methylprednisolone represented by formula (B), prednisolone sodium phosphate represented by formula (C), methylprednisolone acetate represented by formula (D) and methylprednisolone

succinate represented by formula (E);

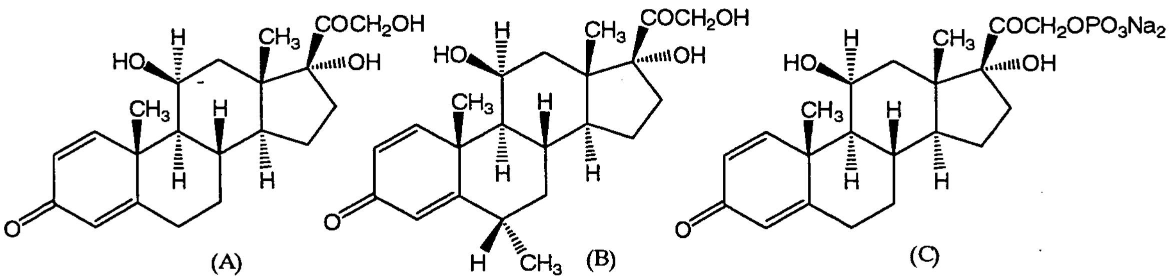

(A) (B) (C)

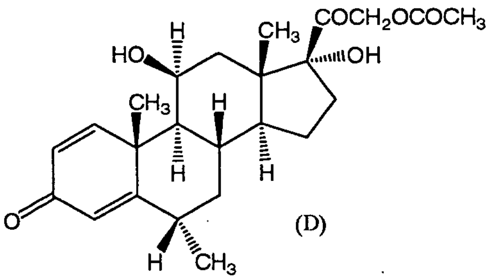

(D)

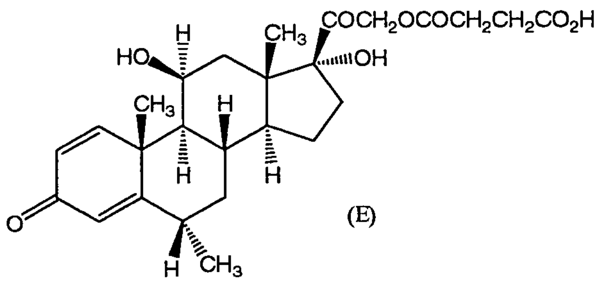

(E)

fluticasone propionate represented by formula (F); beclometasone dipropionate represented by formula (G); budesonide represented by formula (H); flunisolide represented by formula (J); mometasone furoate represented by formula (K);

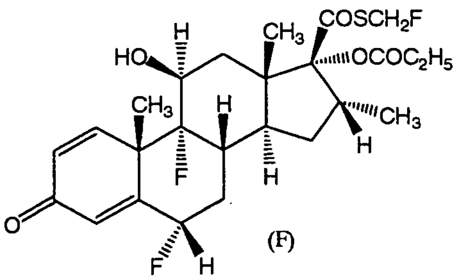

(F)

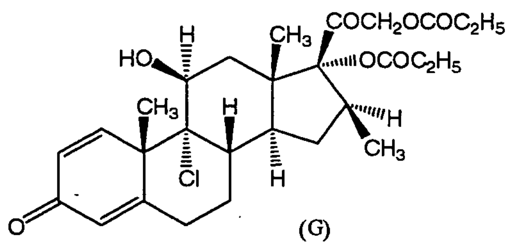

(G)

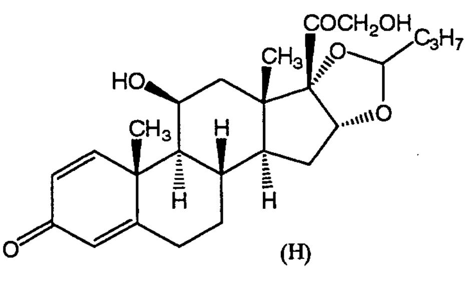

(H)

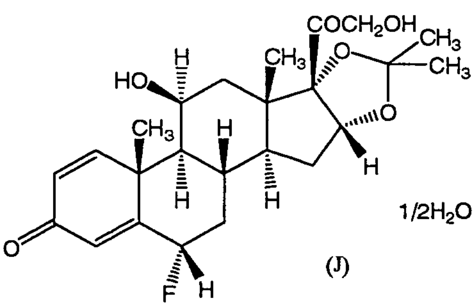

$1/2H_2O$

(J)

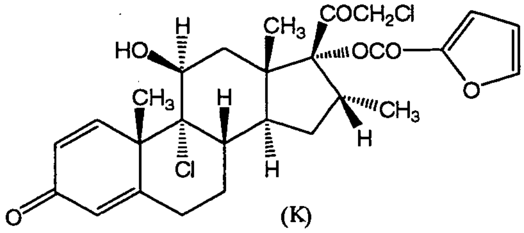

(K)

cortisones such as cortisone acetate represented by formula (L), hydrocortisone represented by formula (M), hydrocortisone succinate represented by formula (N) and fludrocortisone acetate represented by formula (O);

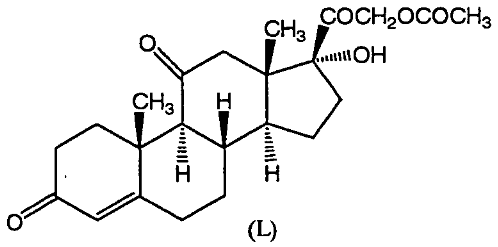

(L)

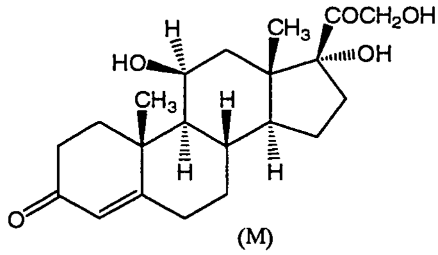

(M)

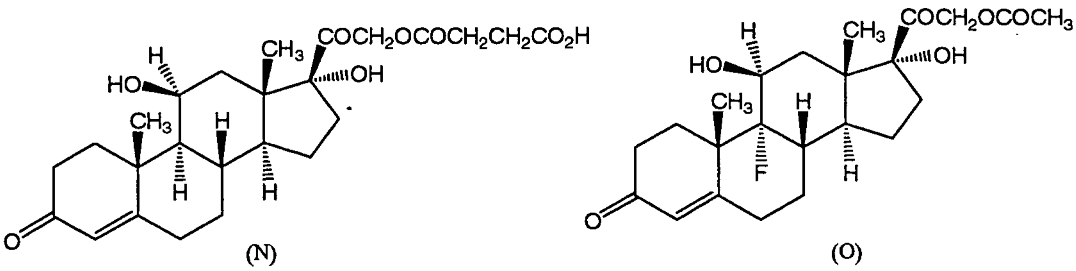

(N) (O)

dexamethazones such as dexamethazone represented by formula (P), dexamethazone propionate represented by formula (Q), dexamethazone sodium phosphate represented by formula (R) and dexamethazone palmitate represented by formula (S);

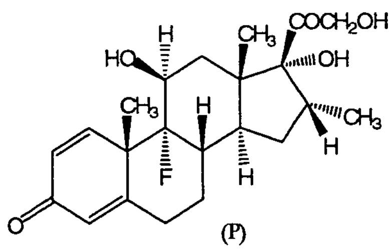

(P)

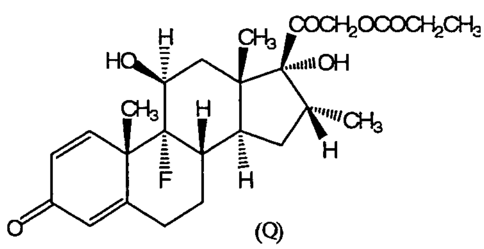

(Q)

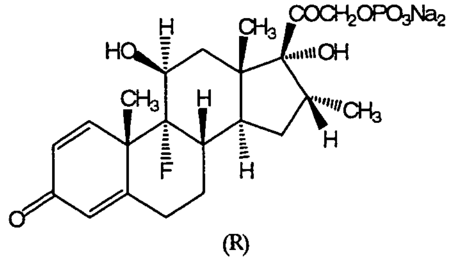

(R)

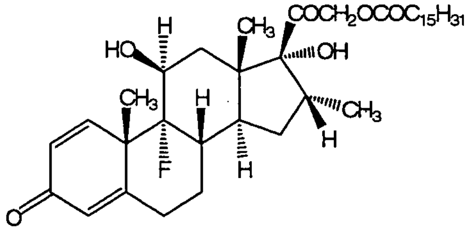

(S)

betametasones such as betametasone represented by formula (T) and betametasone dipropionate represented by formula (U); triamcinolone represented by formula (V); triamcinolone acetonide represented by formula (W); parametasone acetate represented by formula (X); prednisone represented by formula (Y); halopredone acetate and the like. Among these compounds, prednisolone, methylprednisolone, prednisone, dexamethazone, fluticasone propionate, beclometasone dipropionate, budesonide, mometasone furoate and the like are preferred. These compounds may be used alone or in combination.

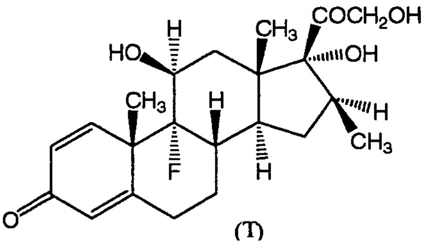
(T)

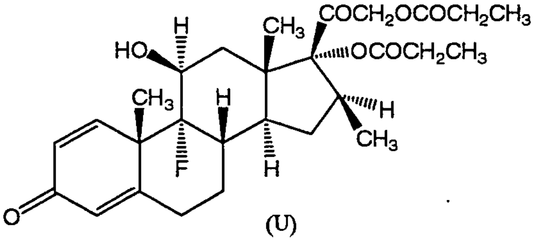
(U)

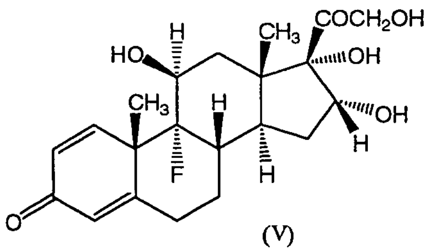
(V)

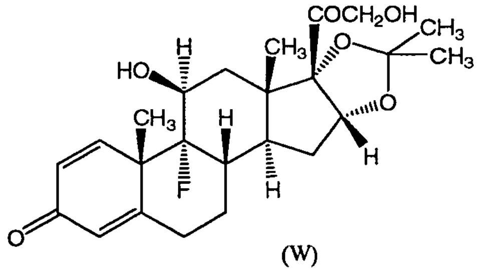
(W)

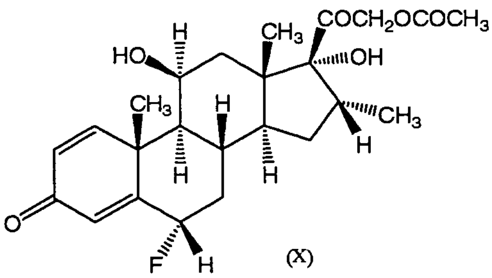
(X)

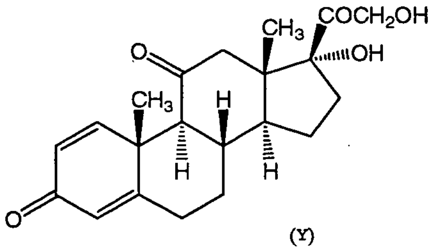
(Y)

These steroid agents may be existed as pharmaceutically acceptable salts (examples of the pharmaceutically acceptable salts include the above-described pharmaceutically acceptable salts of Compound (I) and the like) or hydrates thereof. These pharmaceutically acceptable salts and hydrates can also be used for the pharmaceutical composition, the agent for treating and/or preventing a pulmonary disease, the kit, the kit for treating and/or preventing a pulmonary diseases, and the method for treating and/or preventing a pulmonary disease of the present invention. Also, each of the steroid agents may contain one or more asymmetric carbon(s) and two or more stereoisomers. However, all possible isomers and mixtures thereof can be used for the pharmaceutical composition, the agent for treating and/or preventing a pulmonary disease, the kit, the kit for treating and/or preventing a pulmonary disease, and the method for treating and/or preventing a pulmonary disease of the present invention.

The pharmaceutical composition and the kit of the present invention can be used for, for example, treating pulmonary diseases and the like. More specifically, they can be used for treating pulmonary diseases such as COPD, pulmonary emphysema, chronic bronchitis, ARDS, ALI, asthma, bronchial asthma, acute eosinophilic pneumonia, chronic eosinophilic pneumonia, interstitial pneumonia, pulmonary fibrosis and the like.

The pharmaceutical composition of the present invention or Compound (I) or a pharmaceutically acceptable salt thereof used for treating and/or preventing a pulmonary disease and the steroid agent can be used or administered as a single preparation (a mixture) or as a combination of preparations as long as the preparations are prepared so as to contain each of these active ingredients. In particular, a combination of two or more preparations is preferred. When using or administrating a combination of preparations, these preparations may be used or administered simultaneously or separately with an interval. The preparations are preferably used as a form such as a tablet, an injection or an inhalant

such as a dry powder and an aerosol.

The dose ratio (weight/weight) of Compound (I) or a pharmaceutically acceptable salt thereof to the steroid agent may be appropriately controlled according to the combination with the steroid agent used, the efficacy of the steroid agent used, and the like. Specific example of the dose ratio is 1/50 (Compound (I) or a pharmaceutically acceptable salt thereof/steroid agent) to 50,000/1, preferably 1/30 to 10,000/1, more preferably 1/20 to 5,000/1, and further preferably 1/10 to 1,000/1.

When a combination of preparations is administered, for example, (a) a first component containing Compound (I) or a pharmaceutically acceptable salt thereof and (b) a second component containing the steroid agent are separately prepared in preparations respectively as described above to prepare a kit. And then, the kit can be used for administering the each component to the same object simultaneously or separately with an interval through same route or different routes.

The kit may be formed two or more vessels (for example, vials, bags and the like) and the contents thereof, wherein the vessels are not limited by the materials and the shape as long as it does not cause denaturation of the component of the content due to external temperature or light during preservation and elution of the chemical component from the vessel, and may be used as the form to be able to administrate the above first and second components through different routes (for example, tubes) or the same route. Specific examples include a kit such as tablets, injections, inhalants and the like. The kit of inhalants can be combined with a generally used inhalator such as a metered- dose inhalator (MDI), a powder inhalator or the like. The kit may include one or more inhalators for administering the above first and second components. For example, the kit may comprise a capsule containing a dry powder of the effective dose unit of the above first component and a capsule containing a dry powder of the effective dose unit of the above second component, and together with one or more dry powder inhalator(s) suitable for delivering the dry powder from the capsules. The kit may also include a multi-dose dry powder inhalator (MDPI) comprising a medicine storage part containing the dry powder of the above first component and a medicine storage part containing the dry powder of the above second component.

The method for treating and/or preventing a pulmonary disease of the present invention can be performed by the same method as that of the method for using or administering the above-mentioned pharmaceutical composition or Compound (I) or a pharmaceutically acceptable salt thereof and the steroid agent, which are used for treating and/or preventing a pulmonary disease. Namely, Compound (I) or a pharmaceutically acceptable salt thereof and the steroid agent are prepared in a preparation to contain each of the active ingredients, and administered, for example, as a single preparation or as a combination of preparations, preferably as a combination of two or more preparations. When a combination of preparations is administered, the preparations can be administered simultaneously or separately with an interval or administered using the above-mentioned kit.

Next, the therapeutic effect on a pulmonary disease by simultaneous administrations of Compound (I) or a pharmaceutically acceptable salt thereof and the steroid agent will be described in detail with reference to test examples.

Test Example 1 Inhibitory effect on tumor necrosis factor-α (TNF-α) production

BALB/c mice (male, 7-week-old, Charles River Japan) are intraperitoneally administered with 1 mL of 3 wt/vol % thioglycolate, and the peritoneal effusions are recovered 72 hours later to prepare peritoneal exudative cells. The prepared cells of $5\times10^4$ are inoculated in a 96-well plate and cultured in the presence of lipopolysaccharide (LPS; derived from *E. coli* 055: B5, 1,000 μg/mL) for 6 hours. Then, the culture supernatant is recovered, and the amount of TNF-α in the culture supernatant is measured by an ELISA method. A test compound is dissolved in a dimethylsulfoxide (DMSO) solution of 1 vol/vol% RPMI1640 (DMSO-RPMI solution), and the resultant solution is added simultaneously with LPS (test compound-added group). Also, the group which the DMSO-RPMI solution is added simultaneously with LPS is called solvent-added group, and the group which only the DMSO-RPMI solution is added is called non-added group. The inhibition ratio (%) of TNF-α production is calculated according to the following equation:

$$\text{Inhibition ratio (\%)} = \frac{A - B}{A - C} \times 100$$

wherein A is Solvent-added group, B is Test compound-added group and C is Non-added group. Test Example 2 Inhibitory effect of combined administration of Compound (I) and steroid agent on antigen-induced respiratory eosinophil and neutrophil infiltration in rat.

The test was conducted according to a known method (Pulmonary Pharmacology, vol. 8, pp. 83-89 (1995)).

In the test, 6-week-old male BN rats (Charles River Japan) were used. The rats were placed in aluminum cages each containing 5 to 6 rats in a breeding room at room temperature (19°C to 25°C) and a humidity of 30% to 70% under

illumination for 12 hours a day (7 a.m. to 7 p.m.) and bred by free intake of commercially available feeds and water.

A preparation (Compound (I)-lactose preparation) containing 5 mg of lactose (produced by DVM Corporation) to 1 mg of Compound (I) was prepared, and a suspension for administering Compound (I) was prepared by suspending the Compound (I)-lactose preparation in a 0.125 wt/vol% tyloxapol aqueous solution (ALEVAIRE: registered trade mark, produced by Azwell Inc.) so that the concentration of Compound (I) was 150 μg/mL. Also, a suspension for administering budesonide was prepared by suspending lactose and budesonide (manufactured by Sigma Corporation) in a 0.125 wt/vol% tyloxapol aqueous solution so that the concentrations of lactose and budesonide were 750 μg/mL and 50 μg/mL, respectively. Furthermore, a suspension for combined administration was prepared by suspending budesonide and the Compound (I)-lactose preparation prepared as described above in a 0.125 wt/vol% tyloxapol aqueous solution so that the concentrations of budesonide and Compound (I) were 50 μg/mL and 150 μg/mL, respectively. On the other hand, a solvent for administration was prepared by dissolving lactose in a 0.125 wt/vol% tyloxapol aqueous solution so that the concentration was 7.5 mg/mL.

The rats were subcutaneously administered with 1 mL of a suspension of 1 mg of ovalbumin (OVA, manufactured by Sigma Co.) and 200 mg of aluminum hydroxide (manufactured by Wako Pure Chemicals Industries, Ltd.) suspended in 1 mL of physiological saline (manufactured by Otsuka Pharmaceutical Co., Ltd.). Then, 0.5 mL of a suspension of killed Bordetella pertussis cells ($2x10^{10}$ cells/1 mL physiological saline, manufactured by Kaken Pharmaceutical Co., Ltd.) was intraperitoneally administered to perform active sensitization. Fourteen days after the active sensitization, the rats were placed in a plastic chamber (30x50x30 cm), and an 1 wt/vol% OVA physiological saline solution was nebulized for 10 minutes using an ultrasonic nebulizer (manufactured by Omron Corporation) to perform antigen challenge. Thirty minutes before the antigen challenge, 100 μL of each of the suspensions for administration prepared as described above was intrabronchially administered to each of the right and left bronchi, i.e., administered in a total of 200 μL (medicine-administered group). Similarly, thirty minutes before the antigen challenge, a group which the solvent for administration was intrabronchially administered (solvent-administered group) was prepared. Also, a group which the solvent for administration was intrabronchially administered and a physiological saline was nebulized instead of the 1 wt/vol% OVA physiological saline solution (physiological saline-administered group) was prepared.

Twenty-four hours after the nebulization of the 1 wt/vol% OVA physiological saline solution or the physiological saline, bronchoalveolar lavage (BAL) was carried out. Namely, rats were anesthetized by intraperitoneal administration of pentobarbital (manufactured by Dainippon Pharmaceutical Co., Ltd.), and lavage of the alveolar spaces by physiological saline was carried out through a bronchial cannula to obtain a bronchoalveolar lavage fluid (BALF).

The total number of the leukocytes in BALF was measured with an automatic leukocyte counter (manufactured by Nihon Kohden Corporation). Also, a smear of the cells was prepared and stained with Giemsa, and the numbers of eosinophils and neutrophils were counted by observation with an optical microscope to calculate the ratios of the eosinophils and neutrophils. The numbers of the eosinophils and neutrophils were calculated by multiplying the total number of the leukocytes by the respective ratios of the cells. The inhibition ratios (%) on the increases in the numbers of the eosinophils and neutrophils in BALF were calculated according to the following equations:

$$\text{Inhibition ratio (\%) of number of eosinophils} = \frac{D - E}{D - F} \times 100$$

(wherein D is number of eosinophils in solvent-administered group, E is number of eosinophils in medicine-administered group and F is number of eosinophils in physiological saline-administered group.)

$$\text{Inhibition ratio (\%) of number of neutrophils} = \frac{G - H}{G - I} \times 100$$

(wherein G is number of neutrophils in solvent-administered group, H is number of neutrophils in medicine-administered group and I is number of neutrophils in physiological saline-administered group.)

The results for increases in the number of the eosinophils are shown in Table 1, and the results for increases in the number of the neutrophils are shown in Table 2. Each of the numbers of the eosinophils and neutrophils is shown by mean±standard error.

Table 1

| Administration group | Dose (μg/rat) | | Number of rats | Number of eosinophils ($10^6$/BALF) | Inhibition ratio (%) |
|---|---|---|---|---|---|
| | Compound (I) | Budesonide | | | |
| Physiological saline | - | - | 5 | 0.02±0.01 | - |
| Solvent | - | - | 5 | 0.54±0.22 | - |
| Compound (I) | 30 | - | 5 | 0.26±0.08 | 54 |
| Budesonide | - | 10 | 5 | 0.26±0.06 | 54 |
| Combined administration | 30 | 10 | 4 | 0.16±0.08 | 73 |

Table 2

| Administration group | Dose (μg/rat) | | Number of rats | Number of neutrophils ($10^6$/BALF) | Inhibition ratio (%) |
|---|---|---|---|---|---|
| | Compound (I) | Budesonide | | | |
| Physiological saline | - | - | 5 | 0.11±0.05 | - |
| Solvent | - | - | 5 | 2.34±0.55 | - |
| Compound (I) | 30 | - | 5 | 2.28±0.27 | 3 |
| Budesonide | - | 10 | 5 | 2.50±0.31 | N.E.* |
| Combined administration | 30 | 10 | 4 | 1.58±0.52 | 34 |

*: N.E. No effect

The number of the eosinophils in BALF was increased by antigen challenge to 27 times the number of the eosinophils in BALF of the physiological saline group. The inhibitory effect against increases in the number of eosinophils was exhibited by administering Compound (I) (30 μg/rat) and budesonide (10 μg/rat), respectively. However, the strong inhibitory effect against increases in the number of the eosinophils was observed by combined administration of Compound (I) and budesonide, as compared with sole administration of each of the compounds.

On the other hand, the number of the neutrophils in BALF was increased by antigen challenge to 21 times the number of the eosinophils in BALF in the physiological saline group. The number of neutrophils in BALF was not significantly affected even by solely administering each of Compound (I) (30 μg/rat) and budesonide (10 μg/rat). However, the inhibitory effect against increases in the number of the neutrophils was observed by combined administration of Compound (I) and budesonide.

Asthma and bronchial asthma are diseases characterized by eosinophilic airway inflammation (Clinical Experimental Allergy (Clin. Exp. All.), vol. 32, p. 162 (2002)). It is also known that marked eosinophilic infiltration to the pulmonary tissue and a significant increase in the number of eosinophils in BALF are observed in patients with asthma and bronchial asthma.

On the other hand, many neutrophils are observed in BALF and the sputum obtained from a COPD patient, and the larger the number of the neutrophils in the sputum or bronchial mucosa of a patient, the worse the respiratory obstruction becomes (American Review of Respiratory Disease (Am. Rev. Respir. Dis.), vol. 140, p. 1527 (1989); American Journal of Respiratory and Critical Care Medicine (Am. J. Respir. Crit. Care Med.), vol. 153, p. 530 (1996); and American Journal of Respiratory and Critical Care Medicine (Am. J. Respir. Crit. Care Med.), vol. 158, p. 1277 (1998)).

Therefore, from the results of the above test, it is considered that the combined administration of Compound (I) or a pharmaceutically acceptable salt thereof and the steroid agent is effective in treating and/or preventing pulmonary diseases such as asthma, bronchial asthma, acute eosinophilic inflammation, chronic eosinophilic inflammation, COPD and the like.

On the other hand, a steroid agent exhibits a strong anti-inflammatory effect and is a good medicine as a therapeutic agent for pulmonary diseases such as asthma, bronchial asthma, COPD and the like. However, it has been reported that a steroid agent has an adverse effect on the oral pharynx, bone metabolism and the like, and also causes a severe adverse effect such as disturbance in growth of children (American Journal of Respiratory and Critical Care Medicine (Am. J. Respir. Crit. Care Med.), vol. 157, p. S1 (1998)).

The results of the above test show that the dose of the steroid agent conventionally used for treating pulmonary

diseases such as asthma, bronchial asthma, COPD and the like can be decreased by combined administration of Compound (I) or a pharmaceutically acceptable salt thereof and the steroid agent. In other words, the dose of the steroid agent used for treating pulmonary diseases such as asthma, bronchial asthma, COPD and the like can be decreased by the pharmaceutical composition, the agent for treating and/or preventing a pulmonary disease, the kit, and the kit for treating and/or preventing a pulmonary diseases of the present invention, and it is expected that the effect of the steroid agent conventionally used as a single preparation can be improved, and the adverse effect can be also decreased.

As described above, the pharmaceutical composition or the agent for treating and/or preventing a pulmonary diseases used in the present invention can be used, administered or produced as a single preparation or a combination of preparations as long as they are prepared to contain active ingredients of each of Compound (I) or a pharmaceutically acceptable salt thereof and the steroid agent. The pharmaceutical composition or the agent for treating and/or preventing a pulmonary disease is preferably in a unit dose form suitable for oral administration such as a tablet and the like, or parenteral administration such as an inhalant, an injection and the like. When a combination of preparations is used or administered, the preparations can be administered simultaneously or separately with an interval.

The preparations can be prepared by an ordinary method properly using the active ingredients and pharmaceutically acceptable additives such as a diluent, an excipient, a disintegrator, a lubricant, a binder, a surfactant, water, physiological saline, a vegetal oil solubilizer, an isotonizing agent, a preservative, an antioxidant and the like.

In order to prepare a tablet, an excipient such as lactose, a disintegrator such as starch, a lubricant such as magnesium stearate, a binder such as hydroxypropyl cellulose, a surfactant such as a fatty acid ester, a plasticizer such as glycerin, and the like may be used according to an ordinary method.

In order to prepare an injection, water, physiological saline, a vegetal oil such as soybean oil, any of various solvents, a solubilizer, an isotonizing agent, a preservative, an antioxidant and the like may be used according to an ordinary method.

An inhalant can be produced by a method in which an active ingredient is powdered or liquidized, then mixed with propellants for inhalation or carriers, and the resultant mixture is charged in a suitable inhalation vessel. When the active ingredient is a powder, a general mechanical inhalator can be used, while when the active ingredient is a liquid, an inhalator such as a nebulizer can be used. As the propellants for inhalation, conventionally known agents can be widely used. Examples of thereof include fron compounds such as fron-11, fron-12, fron-21, fron-22, fron-113, fron-114, fron-123, fron-142c, fron-134a, fron-227, fron-C318 and 1,1,1,2-tetrafluoroethane; hydrocarbons such as propane, isobutene and n-butane; ethers such as diethyl ether; and compressed gases such as nitrogen gas and carbon dioxide gas. Furthermore, if necessary, one or more auxiliary component(s) selected from various diluents; preservatives; flavors; and the excipient, the disintegrator, the lubricant, the binder, the surfactant and the plasticizer which are exemplified above; and the like can be added.

When Compound (I) or a pharmaceutically acceptable salt thereof and the steroid agent are used or administered as a combination of preparations for the above-described purpose, the dose and the frequency of administration of each component depend on the administration form and the age, body weight, symptoms and the like of a patient. However, each of Compound (I) or a pharmaceutically acceptable salt thereof and the steroid agent is preferably administered in the daily doses as described below.

In oral administration, for example, the administration of a tablet, Compound (I) or a pharmaceutically acceptable salt thereof and the steroid agent are generally administered simultaneously or separately with an interval in single daily doses or divided daily doses of 0.01 to 1000 mg and 0.01 to 1000 mg, preferably 0.05 to 300 mg and 0.1 to 300 mg, and more preferably 0.5 to 200 mg and 0.5 to 200 mg, respectively, per adult.

In parenteral administration, for example, the administration of an injection or an inhalant, Compound (I) or a pharmaceutically acceptable salt thereof and the steroid agent are generally administered simultaneously or separately with an interval in single daily doses or divided daily doses of 1 μg to 100 mg and 0.01 to 1000 mg, preferably 5 μg to 30 mg and 0.05 to 100 mg, and more preferably 10 μg to 20 mg and 0.1 to 10 mg, respectively, per adult.

When Compound (I) or a pharmaceutically acceptable salt thereof and the steroid agent are used or administered as a single preparation for the above-described purpose, the dose and of the frequency of administration of each component depend on the administration form and the age, body weight, symptoms and the like of a patient. However, Compound (I) and a pharmaceutically acceptable salt thereof and the steroid agent are preferably used or administered in the same daily doses, respectively, as in use or administration of a combination of preparations.

An embodiment of the present invention will be explained below with reference to the following examples. However, the scope of the present invention is not limited to the following examples.

## Best Mode for Carrying Out the Invention

### Example 1: Tablet (Compound (I))

A tablet having the following composition is prepared by an ordinary method. Namely, 40 g of Compound (I),

286.8 g of lactose, and 60 g of potato starch powder are mixed, and 120 g of a 10% aqueous solution of hydroxypropyl cellulose is added to the mixture. The resulting mixture is kneaded by an ordinary method, granulated, dried, and then sized to prepare tablet-making granules. Then, 1.2 g of magnesium stearate is added to the granules, mixed therewith, and tableted with a tableting machine (manufactured by Kikusui Seisakusho, RT-15 model) having a punch of 8 mm in diameter to obtain tablets (each containing 20 mg of the active ingredient).

| Prescription | |
|---|---|
| Compound (I) | 20 mg |
| Lactose | 143.4 mg |
| Potato starch powder | 30 mg |
| Hydroxypropyl cellulose | 6 mg |
| Magnesium stearate | 0.6 mg |
| | 200 mg |

Example 2: Tablet (prednisolone)

A tablet having the following composition is prepared by an ordinary method. Namely, 40 g of prednisolone, 286.8 g of lactose, and 60 g of potato starch powder are mixed, and 120 g of a 10% aqueous solution of hydroxypropyl cellulose is added to the mixture. The resulting mixture is kneaded by an ordinary method, granulated, dried, and then sized to prepare tablet-making granules. Then, 1.2 g of magnesium stearate is added to the granules, mixed therewith, and tableted with a tableting machine (manufactured by Kikusui Seisakusho, RT-15 model) having a punch of 8 mm in diameter to obtain tablets (each containing 20 mg of the active ingredient).

| Prescription | |
|---|---|
| Prednisolone | 20 mg |
| Lactose | 143.4 mg |
| Potato starch powder | 30 mg |
| Hydroxypropyl cellulose | 6 mg |
| Magnesium stearate | 0.6 mg |
| | 200 mg |

Example 3: Tablet (single preparation containing Compound (I) and prednisolone)

A tablet having the following composition is prepared by an ordinary method. Namely, 40 g of Compound (I), 40 g of prednisolone, 246.8 g of lactose, and 60 g of potato starch powder are mixed, and 120 g of a 10% aqueous solution of hydroxypropyl cellulose is added to the mixture. The resulting mixture is kneaded by an ordinary method, granulated, dried, and then sized to prepare tablet-making granules. Then, 1.2 g of magnesium stearate is added to the granules, mixed therewith, and tableted with a tableting machine (manufactured by Kikusui Seisakusho, RT-15 model) having a punch of 8 mm in diameter to obtain tablets (each containing 20 mg of Compound (I) and 20 mg of prednisolone).

| Prescription | |
|---|---|
| Compound (I) | 20 mg |
| Prednisolone | 20 mg |
| Lactose | 123.4 mg |
| Potato starch powder | 30 mg |
| Hydroxypropyl cellulose | 6 mg |
| Magnesium stearate | 0.6 mg |
| | 200 mg |

Example 4: Injection (Compound (I))

An injection having the following composition is prepared by an ordinary method. Namely, 1 g of Compound (I) is dissolved in purified soybean oil, and 12 g of purified egg-yolk lecithin and 25 g of injectable glycerin are added to the solution. The resulting mixture is kneaded and emulsified with 1000 mL of distilled water for injection by an ordinary

method. The resulting dispersion is aseptically filtered through a 0.2 μm disporsable membrane filter, and then 2 mL of the filtrate is aseptically charged in each glass vial to obtain an injection (containing 2 mg of the active ingredient per vial).

| Prescription | |
|---|---|
| Compound (I) | 2 mg |
| Purified soybean oil | 200 mg |
| Purified egg-yolk lecithin | 24 mg |
| Injectable glycerin | 50 mg |
| Distilled water for injection | 1.72 mL |
| | 2.00 mL |

Example 5: Injection (methylprednisolone)

An injection having the following composition is prepared by an ordinary method. Namely, 1 g of methylprednisolone is dissolved in purified soybean oil, and 12 g of purified egg-yolk lecithin and 25 g of injectable glycerin are added to the solution. The resulting mixture is kneaded and emulsified with 1000 mL of distilled water for injection by an ordinary method. The resulting dispersion is aseptically filtered through a 0.2 μm disporsable membrane filter, and then 2 mL of the filtrate is aseptically charged in each glass vial to obtain an injection (containing 2 mg of the active ingredient per vial).

| Prescription | |
|---|---|
| Methylprednisolone | 2 mg |
| Purified soybean oil | 200 mg |
| Purified egg-yolk lecithin | 24 mg |
| Injectable glycerin | 50 mg |
| Distilled water for injection | 1.72 mL |
| | 2.00 mL |

Example 6: Injection (single preparation containing Compound (I) and methylprednisolone)

An injection having the following composition is prepared by an ordinary method. Namely, 1 g of Compound (I) and 1 g of methylprednisolone are dissolved in purified soybean oil, and 12 g of purified egg-yolk lecithin and 25 g of injectable glycerin are added to the solution. The resulting mixture is kneaded and emulsified with 1000 mL of distilled water for injection by an ordinary method. The resulting dispersion is aseptically filtered through a 0.2 μm disporsable membrane filter, and then 2 mL of the filtrate is aseptically charged in each glass vial to an obtain an injection (containing 2 mg of Compound (I) and 2 mg of methylprednisolone per vial).

| Prescription | |
|---|---|
| Compound (I) | 2 mg |
| Methylprednisolone | 2 mg |
| Purified soybean oil | 200 mg |
| Purified egg-yolk lecithin | 24 mg |
| Injectable glycerin | 50 mg |
| Distilled water for injection | 1.72 mL |
| | 2.00 mL |

Example 7: Dry powder inhalant (Compound (I))

A dry powder inhalant having the following composition is prepared by an ordinary method. Namely, Compound (I) is pulverized using a jet mill (volume-average particle size: 5 to 20 μm). The pulverized product of Compound (I) and lactose (Pharmatose 325M; registered trade mark, DMV) are mixed at a weight ratio of 1:5 to obtain a dry powder preparation. The dry powder preparation can be administered by a common dry powder inhalator.

| Prescription | |
|---|---|
| Compound (I) | 16.7 mg |
| Lactose | 83.3 mg |
| | 100 mg |

Example 8: Dry power inhalant (budesonide)

A dry powder inhalant having the following composition is prepared by an ordinary method. Namely, budesonide is pulverized using a jet mill (volume-average particle size: 5 to 20 μm). The pulverized product of budesonide and lactose (Pharmatose 325M; registered trade mark, DMV) are mixed at a weight ratio of 1:10 to obtain a dry powder preparation. The dry powder preparation can be administered by a common dry powder inhalator.

| Prescription | |
|---|---|
| Budesonide | 9.1 mg |
| Lactose | 90.9 mg |
| | 100 mg |

Example 9: Dry powder inhalant (single preparation containing Compound (I) and budesonide)

A dry powder inhalant having the following composition is prepared by an ordinary method. Namely, Compound (I) and budesonide are pulverized using a jet mill (volume-average particle sizes: 5 to 20 μm), respectively. The pulverized products of each Compound (I), budesonide and lactose (Pharmatose 325M; registered trade mark, DMV) are mixed at a weight ratio of 2:1:10 to obtain a dry powder preparation. The dry powder preparation can be administered by a common dry powder inhalator.

| Prescription | |
|---|---|
| Compound (I) | 15.4 mg |
| Budesonide | 7.7 mg |
| Lactose | 76.9 mg |
| | 100 mg |

Industrial Applicability

The present invention provides a pharmaceutical composition, which comprises 7-[2-(3,5-dichloro-4-pyridyl)-1-oxoethyl]-4-methoxy-spiro[1,3-benzodioxol-2,1'-cyclopentane] or a pharmaceutically acceptable salt thereof and a steroid agent, and the like.

## Claims

**1.** A pharmaceutical composition, which comprises (a) 7-[2-(3,5-dichloro-4-pyridyl)-1-oxoethyl]-4-methoxy-spiro[1,3-benzodioxol-2,1'-cyclopentane] represented by formula (I)

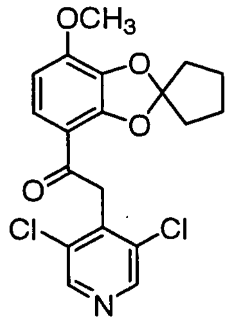

( I )

or a pharmaceutically acceptable salt thereof and (b) a steroid agent.

**2.** The pharmaceutical composition according to claim 1, wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

**3.** An agent for treating and/or preventing a pulmonary disease, which comprises (a) 7-[2-(3,5-dichloro-4-pyridyl)-1-oxoethyl]-4-methoxy-spiro[1,3-benzodioxol-2,1'-cyclopentane] represented by formula (I)

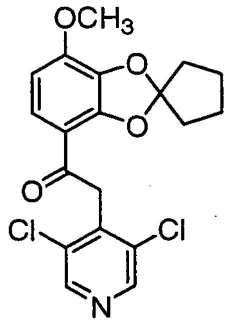

( I )

or a pharmaceutically acceptable salt thereof and (b) a steroid agent as active ingredients, wherein the active ingredients are administered simultaneously or separately with an interval.

**4.** The agent for treating and/or preventing a pulmonary disease according to claim 3, wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

**5.** The agent for treating and/or preventing a pulmonary disease according to claim 3 or 4, wherein the pulmonary disease is that selected from the group consisting of chronic obstructive pulmonary diseases (COPD), pulmonary emphysema, chronic bronchitis, chronic/acute respiratory distress syndrome (ARDS), acute lung injury (ALI), asthma, bronchial asthma, acute eosinophilic pneumonia and chronic eosinophilic pneumonia.

**6.** The agent for treating and/or preventing a pulmonary disease according to claim 3 or 4, wherein the pulmonary disease is that selected from the group consisting of asthma, bronchial asthma, acute eosinophilic pneumonia and chronic eosinophilic pneumonia.

**7.** The agent for treating and/or preventing a pulmonary disease according to claim 3 or 4, wherein the pulmonary disease is COPD.

**8.** A kit which comprises (a) a first component containing 7-[2-(3,5-dichloro-4-pyridyl)-1-oxoethyl]-4-methoxy-spiro[1,3-benzodioxol-2,1'-cyclopentane] represented by formula (I)

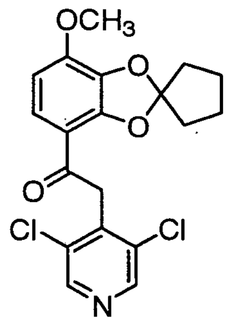

(I)

or a pharmaceutically acceptable salt thereof and (b) a second component containing a steroid agent.

**9.** The kit according to claim 8, wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

**10.** A kit for treating and/or preventing a pulmonary disease, which comprises (a) a first component containing 7-[2-(3,5-dichloro-4-pyridyl)-1-oxoethyl]-4-methoxy-spiro[1,3-benzodioxol-2,1'-cyclopentane] represented by formula (I)

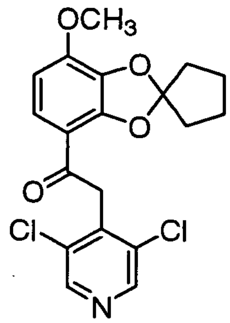

(I)

or a pharmaceutically acceptable salt thereof and (b) a second component containing a steroid agent.

**11.** The kit for treating and/or preventing a pulmonary disease according to claim 10, wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

**12.** The kit for treating and/or preventing a pulmonary disease according to claim 10 or 11, wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.

**13.** The kit for treating and/or preventing a pulmonary disease according to claim 10 or 11, wherein the pulmonary disease is that selected from the group consisting of asthma, bronchial asthma, acute eosinophilic pneumonia and chronic eosinophilic pneumonia.

**14.** The kit for treating and/or preventing a pulmonary disease according to claim 10 or 11, wherein the pulmonary disease is COPD.

**15.** 7-[2-(3,5-Dichloro-4-pyridyl)-1-oxoethyl]-4-methoxy-spiro[1,3-benzodioxol-2,1'-cyclopentane] represented by formula (I)

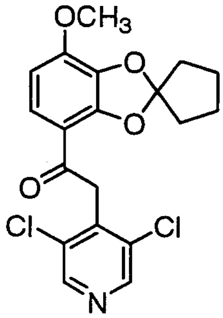

( I )

or a pharmaceutically acceptable salt thereof, which is administered with a steroid agent simultaneously or separately with an interval.

16. 7-[2-(3,5-Dichloro-4-pyridyl)-1-oxoethyl]-4-methoxy-spiro[1,3-benzodioxol-2,1'-cyclopentane] or a pharmaceutically acceptable salt thereof according to claim 15, wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

17. A pharmaceutical composition, which comprises 7-[2-(3,5-dichloro-4-pyridyl)-1-oxoethyl]-4-methoxy-spiro[1,3-benzodioxol-2,1'-cyclopentane] represented by formula (I)

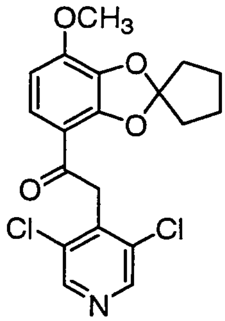

( I )

or a pharmaceutically acceptable salt thereof as an active ingredient which is administered with a steroid agent simultaneously or separately with an interval.

18. The pharmaceutical composition according to claim 17, wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

19. A method for treating and/or preventing a pulmonary disease, which comprises administering (a) 7-[2-(3,5-dichloro-4-pyridyl)-1-oxoethyl]-4-methoxy-spiro[1,3-benzodioxol-2,1'-cyclopentane] represented by formula (I)

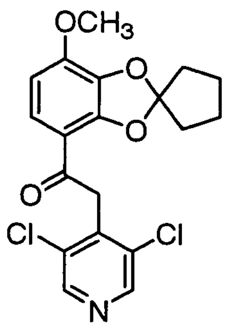

(I)

or a pharmaceutically acceptable salt thereof and (b) a steroid agent simultaneously or separately with an internal.

**20.** The method for treating and/or preventing a pulmonary disease according to claim 19, wherein the steroid agent is a compound selected from the group consisting of prednisolone, methylprednisolone, prednisone, dexamethasone, fluticasone propionate, beclomethasone propionate, budesonide, flunisolide and mometasone furancarboxylate.

**21.** The method for treating and/or preventing a pulmonary disease according to claim 19 or 20, wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.

**22.** The method for treating and/or preventing a pulmonary disease according to claim 19 or 20, wherein the pulmonary disease is that selected from the group consisting of asthma, bronchial asthma, acute eosinophilic pneumonia and chronic eosinophilic pneumonia.

**23.** The method for treating and/or preventing a pulmonary disease according to claim 19 or 20, wherein the pulmonary disease is COPD.

**24.** A method for treating and/or preventing a pulmonary disease, which comprises administering the pharmaceutical composition according to claim 1 or 2.

**25.** The method for treating and/or preventing a pulmonary disease according to claim 24, wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.

**26.** The method for treating and/or preventing a pulmonary disease according to claim 24, wherein the pulmonary disease is that selected from the group consisting of asthma, bronchial asthma, acute eosinophilic pneumonia and chronic eosinophilic pneumonia.

**27.** The method for treating and/or preventing a pulmonary disease according to claim 24, wherein the pulmonary disease is COPD.

**28.** Use of (a) and (b) according to claim 1 or 2 for manufacture of an agent for treating and/or preventing a pulmonary disease.

**29.** Use according to claim 28, wherein the pulmonary disease is that selected from the group consisting of COPD, pulmonary emphysema, chronic bronchitis, ARDS, ALI, asthma, bronchial asthma, acute eosinophilic pneumonia and chronic eosinophilic pneumonia.

**30.** Use according to claim 28, wherein the pulmonary disease is that selected from the group consisting of asthma, bronchial asthma, acute eosinophilic pneumonia and chronic eosinophilic pneumonia.

**31.** Use according to claim 28, wherein the pulmonary disease is COPD.

**INTERNATIONAL SEARCH REPORT**

International application No.
PCT/JP2004/004623

A. CLASSIFICATION OF SUBJECT MATTER
Int.Cl[7] A61K31/443, 31/575, 31/58, A61P11/00, 11/06//C07D405/06, C07J5/00, 7/00, 17/00, 31/00, 71/00

According to International Patent Classification (IPC) or to both national classification and IPC

B. FIELDS SEARCHED

Minimum documentation searched (classification system followed by classification symbols)
Int.Cl[7] A61K31/00-31/80, A61P1/00-43/00, C07D405/00-405/14, C07J1/00-75/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2004 |
| Kokai Jitsuyo Shinan Koho | 1971-2004 | Toroku Jitsuyo Shinan Koho | 1994-2004 |

Electronic data base consulted during the international search (name of data base and, where practicable, search terms used)
MEDLINE(STN), EMBASE(STN), BIOSIS(STN), BIOTECHABS(STN), CAplus(STN), REGISTRY(STN), WPI(DIALOG), JSTPLUS(JOIS), JMEDPLUS(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y | WO 96/36624 A1 (Kyowa Hakko Kogyo Co., Ltd.), 21 November, 1996 (21.11.96), Example 140; description; page 1, line 4 to page 3, line 23 | 15,16<br>1-14,17,18, 28-31 |
| P,X<br>P,Y | WO 03/066044 A1 (BOERINGER INGELHEIM PHARM GMBH & CO., KG), 14 August, 2003 (14.08.03), Example 140; description; page 26, line 26 to page 27, line 2 | 15,16<br>1-14,17,18, 28-31 |
| P,X<br>P,Y | WO 2004/005276 A1 (Kyowa Hakko Kogyo Co., Ltd.), 15 January, 2004 (15.01.04) | 15,16<br>1-14,17,18, 28-31 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>17 June, 2004 (17.06.04) | Date of mailing of the international search report<br>13 July, 2004 (13.07.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/004623

C (Continuation). DOCUMENTS CONSIDERED TO BE RELEVANT

| Category* | Citation of document, with indication, where appropriate, of the relevant passages | Relevant to claim No. |
|---|---|---|
| Y | WO 01/32127 A1 (SMITHKLINE BEECHAM CORP.), 10 May, 2001 (10.05.01), Claims; examples | 1-18,28-31 |
| Y | WO 01/57036 A1 (PFIZER PRODUCTS, INC.), 09 August, 2001 (09.08.01), Claims; page 119, line 18 to page 132, line 13 | 1-18,28-31 |
| Y | WO 01/64639 A2 (MERCK FROSST CANADA & CO.), 07 September, 2001 (07.09.01), Claims; page 27, line 14 to page 29, the last line | 1-18,28-31 |
| A | Yuji YOSHIYAMA, "Jikan Chiryo e Mukete", Pharmacia, 1998, 34(6), pages 573 to 578 | 1-18,28-31 |

Form PCT/ISA/210 (continuation of second sheet) (January 2004)

**INTERNATIONAL SEARCH REPORT**
Information on patent family members

International application No.
PCT/JP2004/004623

| | | |
|---|---|---|
| WO 96/36624 A1 | 1996.11.21 | AU 9657029 A<br>NO 9700151 A<br>EP 771794 A1<br>KR 97704724 A<br>AU 705690 B<br>US 2002/0128290 A1<br>US 6514996 B2<br>CN 1154697 A<br>US 6716987 B1 |
| WO 03/066044 A1 | 2003.08.14 | DE 10205274 A1<br>US 2003/0203918 A1<br>AU 2003205717 A1 |
| WO 2004/005276 A1 | 2004.01.15 | (Family: none) |
| WO 01/32127 A1 | 2001.05.10 | AU 200113575 A<br>BR 200015270 A<br>NO 200202057 A<br>EP 1225866 A2<br>SK 200200759 A3<br>KR 2002057988 A<br>HU 200203152 A2<br>CN 1387404 A<br>JP 2003-513028 A<br>CZ 200201512 A3<br>ZA 200203435 A<br>MX 2002004350 A1 |
| WO 01/57036 A1 | 2001.08.09 | AU 200127002 A<br>NO 200203613 A<br>EP 1252158 A1<br>BR 200107964 A<br>KR 2002072299 A<br>HU 200204262 A2<br>CN 1404481 A<br>JP 2003-522176 A<br>CZ 200202410 A3<br>US 2003/0186989 A1<br>ZA 200206033 A<br>MX 2002007419 A1<br>SK 200201014 A3<br>NZ 519547 A |
| WO 01/64639 A2 | 2001.09.07 | AU 200139051 A<br>US 2002/0068756 A1<br>US 6436965 B1<br>EP 1263728 A2<br>JP 2003-525273 A |

Form PCT/ISA/210 (patent family annex) (January 2004)

**INTERNATIONAL SEARCH REPORT**

International application No.
PCT/JP2004/004623

**Box No. II Observations where certain claims were found unsearchable (Continuation of item 2 of first sheet)**

This international search report has not been established in respect of certain claims under Article 17(2)(a) for the following reasons:

1. [X] Claims Nos.: 19-27
because they relate to subject matter not required to be searched by this Authority, namely:
The inventions as set forth in claims 19-27 pertain to methods for treatment of the human body by therapy. (Article 17(2)(a)(i) of the PCT, Rule 39.1(iv) of the Regulations under the PCT)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**

International application No.
PCT/JP2004/004623

With respect to claims 1, 3, 5-8, 10, 12-15, 17, and 28-31

The claims include "a steroid agent" as an active ingredient of the medicine. However, no definition is given therein on as to what chemical structure among those having a steroid skeleton may be specifically possessed by the agent. This is not considered to be obvious to persons skilled in the art. Even when the statements in the description are taken into account, the compounds specifically shown are limited to the scope of the compounds enumerated in claim 2. No special statement is given on the other compounds.

In view of this, it is not considered that the description is clear and sufficient in such a degree that the subject matters of those claims can be carried out and that the statements in the claims are supported by the description. Consequently, the claims and the statements in the description do not comply with the requirements in Articles 5 and 6 of the PCT.

Since the description and the claims of this application do not comply with the given requirements, a search for making an international search report was made through prior-art documents in a rational range which had been limited based on the statements in the description.

Form PCT/ISA/210 (extra sheet) (January 2004)